# EUROPEAN PATENT APPLICATION

(11) **EP 1 325 911 A1**
(43) Date of publication of application: **09.07.2003**
(21) Application number: 03290021.9
(22) Date of filing: 06.01.2003
(51) Int. Cl.: C07D 209/08, C07D 209/12

(54) **Method for preparing 3-methylindole derivatives through hydrogenation**

(30) Priority: 07.01.2002 KR 2002000734
(71) Applicant: SK Corporation, Seoul 110-110 (KR)
(72) Inventor: Kwak, Byong-Sung, Yusung-gu, Daejeon 305-761 (KR); Kim, Tae-Yun, Yusung-gu, Daejeon 305-728 (KR); Kim, Jin-Woong, Yusung-gu, Daejeon 305-761 (KR); Lee, Sang-II, yusung-gu, Daejeon 305-729 (KR)
(74) Representative: Santarelli

(57) **Abstract**

There is disclosed a preparation method of 3-methylindole derivatives by reducing the aldehyde moiety in indole-3-carboxaldehyde derivatives in the presence of the copper or copper oxide-based hydrogenation catalyst. This process is performed in solvent under the condition of 50-300 °C and 50-1,000 psig in the presence of a solvent with a hydrogen atmosphere. The method is advantageous in the economic aspects because of the employment of a relatively inexpensive hydrogenation catalyst and the easy regeneration thereof. Further, this process is environmentally safe because the intended addition of the sulfur components is not required therefor.

## Description

### TECHNICAL FIELD

The present invention pertains to preparation of 3-methylindole derivatives through hydrogenation. More specifically, the present invention is directed to a method for economically preparing 3-methylindole derivatives from indole-3-carboxaldehyde compounds through the aldehyde-reduction path in the presence of the relatively inexpensive hydrogenation metal catalyst.

### PRIOR ART

3-methylindole derivatives are useful as chemical intermediates in the synthesis of various pharmaceutical drugs. In particular, representative examples of the pharmaceutical compounds are disclosed in EP 0 199 543 A and EP 0 220 066 A, and other compounds including of 3-indoletryptophan, serotonin or melatonin as a main component.

For example, 3-methylindole derivatives are prepared by alkylating indole compounds at the 3-position thereof with alkyl halides, thereby yielding 3-methylindole compounds. However, this process is relatively complicated and difficult to perform, through which by-products alkylated at 1-position and/or 2-position are produced in large amounts.

Alternatively, 3-methylindole compounds may be prepared by reducing indole-3-carboxaldehyde compounds in the presence of the hydrogenation catalyst.

In this regard, it has been known in the art that 3-methylindole is produced by reducing indole-3-carboxaldehyde in the presence of the lithium aluminum hydride catalyst (Can. J. Chem. 31. 775 (1953)). However, the above process is disadvantageous in that the expensive lithium aluminum hydride catalyst should be employed in stoichiometric amounts based on the indole-3-aldehyde, thus increasing a production cost. As well, by-products are produced in large quantities.

Further, the preparation of 3-methylindole using t-butyllithium has been reported (J. Org. Chem. 59, 10 (1994)). However, this method is undesirable in the economic aspects since an expensive butyllithium is used and the reaction should be conducted at temperature as low as about -78 °C.

In Japanese Patent Laid-open No. Sho. 63-297363, there is disclosed a method of preparing 3-methylindole using palladium or Raney nickel among the Groups VIB and VIIIB as a hydrogenation catalyst from indole-3-carboxaldehyde. According to the above prior art, a sulfur component is preferably used in order to increase the selectivity of 3-methylindole. But, the catalyst is so readily poisoned due to the sulfur components that the catalytic activity is drastically lowered as the reaction goes on. Further, the palladium/carbon (Pd/C) catalyst, which is mentioned as being suitable in the above reference, is relatively expensive and there's need of intended addition of sulfur component which is environmentally unfavorable.

### DISCLOSURE OF THE INVENTION

Leading to the present invention, the intensive and thorough research into preparation methods of 3-methylindole derivatives, carried out by the present inventors aiming to solve the problems encountered in the prior arts, resulted in the finding of a method of preparing 3-methylindole derivatives through reduction of indole-3-carboxaldehyde derivatives in the presence of a copper- or copper oxide-based catalyst which is an inexpensive hydrogenation catalyst.

Therefore, it is an object of the present invention to provide a method of preparing 3-methylindole derivatives through reduction of indole-3-carboxaldehyde derivatives at high yields without intended addition of sulfur components.

It is another object of the present invention is to provide a method of preparing 3-methylindole derivatives, which is desirable in the economic and environmental aspects.

In accordance with the present invention, there is provided a method for preparing 3-methylindole derivatives, in which indole-3-carboxaldehyde derivatives having the following formula 1 are subjected to reduction in solvent using hydrogenation catalyst under the condition of a temperature of 50-300 °C and a pressure of 50-1,000 psig with hydrogen atmosphere to give 3-methylindole derivatives having the following formula 2, the hydrogenation catalyst comprising copper or copper oxide and inorganic oxide selected from the group consisting of silica, alumina, zirconia, titania, zinc oxide, chromium oxide, and combinations thereof:

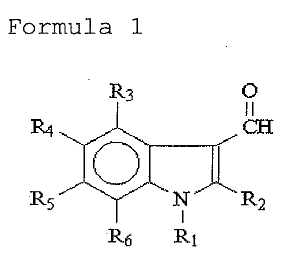

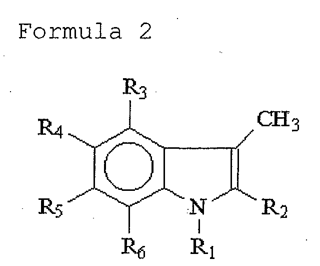

Wherein R₁, R₂, R₃, R₄, R₅ and R₆, which are the same or different, each represents a hydrogen atom, a halogen atom, a hydroxy group, an alkyl group of 1-6 carbon atoms, an aryl group of 6-12 carbon atoms, or an alkoxy group of 1-12 carbon atoms.

### BRIEF DESCRIPTION OF THE DRAWING

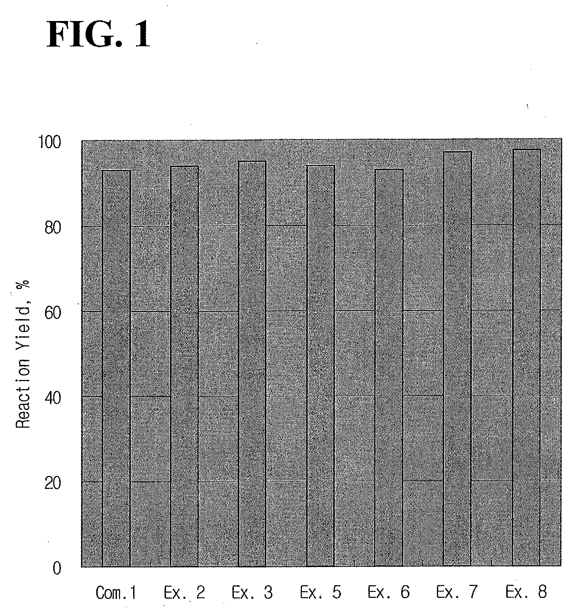

Fig. 1 is a graph showing each of the reaction yields of the products obtained by Comparative Example 1 and Examples 1-8.

### BEST MODES FOR CARRYING OUT THE INVENTION

According to the present invention, there are used indole-3-carboxaldehyde derivatives having the following Formula 1 as starting compounds or reactant:

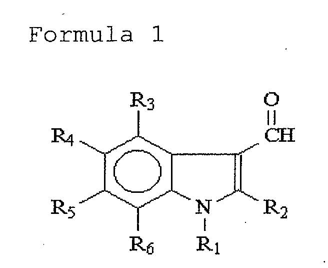

The aldehyde moiety in such starting compounds is subjected to reduction under hydrogen atmosphere in the presence of a particular hydrogenation catalyst which is desirable in terms of yield and selectivity of 3-methylindole derivatives having the following formula 2 as well as production cost: 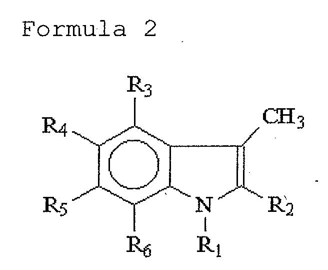 wherein R₁, R₂, R₃, R₄, R₅ and R₆, which are the same or different, each represents a hydrogen atom, a halogen atom, a hydroxy group, an alkyl group of 1-6 carbon atoms, an aryl group of 6-12 carbon atoms, or an alkoxy group of 1-12 carbon atoms.

The hydrogenation catalyst is a copper- and copper oxide-based catalyst, in which inorganic oxide is combined in the form of a support, a binder or a co-catalyst. Suitable is cupric chloride, cupric nitrate, cupric sulfate or copper acetate as a copper precursor. It is noted that the amount of copper in the catalyst should be adjusted in the proper range considering that too low copper amount results in decreased reaction activity, while too higher copper amount hardly increases the reaction rate beyond the limited level, thus causing an economical disadvantage. In this regard, it is preferred that the copper component is contained at an amount of 3-50 wt% on basis of the catalyst.

The inorganic oxide used in the catalyst is preferably selected from the group consisting of silica, alumina, zirconia, titania, zinc oxide and chromium oxide. The inorganic oxide may be used alone or in combinations thereof.

The using amount of the catalyst should be controlled to achieve desirable yield and selectivity of 3-methylindole derivatives while maintaining economic benefits, as described earlier. Preferably, the catalyst is used in the amount of 0.5-200 parts by weight, and more preferably in the amount of 1-100 parts by weight, based on 100 parts by weight of the indole-3-carboxaldehyde derivatives.

In accordance with the present invention, the reduction of indole-3-carboxaldehyde derivatives to 3-methylindole derivatives is carried out in solvent. Accordingly, it is preferred to use a solvent which does not react with the reactant and the product, and is capable of dissolving the reactant and the product therein. Such a solvent is exemplified by polar compound such as ether (e.g. diglyme and dioxane), alcohol (e.g. methanol, ethanol and propanol), and mixtures thereof.

The process according to the present invention may be performed by a continuous fixed-bed reaction or a batch-type reaction.

In accordance with the present invention, the process is carried out at 50-300 °C, preferably at 80-250 °C, and under pressure of 50-1,000 psig, and preferably of 100-700 psig with hydrogen atmosphere. As such, in cases where the reaction conditions fall outside of the above ranges, the activity is decreased or the side-reactions occur.

In the light of the foregoing, the method of the present invention is advantageous in terms of an economical preparation of 3-methylindolé compounds through an environmentally favorable process due to use of the inexpensive hydrogenation catalyst and reusability of the used catalyst without intended addition of a sulfur component.

Having generally described this invention, a further understanding can be obtained by reference to certain specific examples which are provided herein for purposes of illustration only and are not intended to be limiting unless otherwise specified.

### COMPARATIVE EXAMPLE 1

Under a hydrogen atmosphere, 8 g of indole-3-carboxaldehyde (sulfur content: 180 ppm), 30 g of diglyme and 0.8 g of palladium-carbon (palladium content: 5 wt%) catalyst were introduced into a batch-type reactor, and reacted at 140 °C under a hydrogen pressure of 250 psig for 8 hours.

When the sample collected at regular time intervals reached 100% in conversion efficiency, as analyzed by high performance gas chromatography, the reaction was stopped and the reactor temperature was decreased, to prepare a 3-methylindole at a reaction yield of 93%.

### EXAMPLE 1

Under the hydrogen atmosphere, 8 g of indole-3-carboxaldehyde (sulfur content: 300 ppm), 30 g of diglyme and 0.8 g of Cu/SiO₂ (Cu content: 20 wt%) catalyst were introduced into a batch-type reactor, and reacted at 170 °C under a hydrogen pressure of 400 psig for 6 hours, to prepare 3-methylindole at a reaction yield of 93%.

### EXAMPLE 2

Under the hydrogen atmosphere, 8 g of indole-3-carboxaldehyde (sulfur content: 180 ppm), 30 g of diglyme and 0.9 g of CuO/SiO₂ (CuO content: 25 wt%) catalyst were introduced into the batch-type reactor, and reacted at 180 °C under a hydrogen pressure of 400 psig for 6 hours, to prepare 3-methylindole at a reaction yield of 94%.

### EXAMPLE 3

Under the hydrogen atmosphere, 8 g of indole-3-carboxaldehyde (sulfur content: 5 ppm or less), 30 g of diglyme and 0.9 g of CuO/SiO₂ (CuO content: 25wt%) catalyst were introduced into the batch-type reactor, and reacted at 160 °C under a hydrogen pressure of 400 psig for 5 hours, to prepare 3-methylindole at a reaction yield of 95%.

### EXAMPLE 4

Under the hydrogen atmosphere, the CuO/SiO₂ catalyst, which was already used in Example 3, was regenerated through the filtration and recovered. Thereafter, such a recovered catalyst was reused for the reduction of indole-3-carboxaldehyde in the same manner as in Example 3, to prepare 3-methylindole at a reaction yield of 93%.

### EXAMPLE 5

Under the hydrogen atmosphere, 8 g of indole-3-carboxaldehyde (sulfur content: below 5 ppm), 30 g of diglyme and 0.8 g of CuO-ZnO (CuO content: 30 wt%) catalyst were introduced into the batch-type reactor, and reacted at 170 °C under a hydrogen pressure of 400 psig for 6 hours, to prepare 3-methylindole at a reaction yield of 94%.

### EXAMPLE 6

Under the hydrogen atmosphere, 8 g of indole-3-carboxaldehyde (sulfur content: below 5 ppm), 30 g of diglyme and 0.8 g of copper chromite (CuO content: 33 wt%) catalyst were introduced into the batch-type reactor, and reacted at 165 °C under a hydrogen pressure of 400 psig for 7 hours, to prepare 3-methylindole at a reaction yield of 93%.

### EXAMPLE 7

Under the hydrogen atmosphere, 8 g of indole-3-carboxaldehyde, 30 g of diglyme and 1.4 g of copper chromite catalyst (Cu content: 28 wt%) reduced at 200°C for 3 hours were introduced into the batch-type reactor, and reacted at 135 °C under a hydrogen pressure of 430 psig for 4 hours, to prepare 3-methylindole at a reaction yield of 97%.

### EXAMPLE 8

Under the hydrogen atmosphere, 8 g of indole-3-carboxaldehyde, 30 g of diglyme and 1.6 g of Copper-Silica catalyst (Cu content: 20 wt%) reduced at 200°C for 3 hours were introduced into the batch-type reactor, and reacted at 130 °C under a hydrogen pressure of 400 psig for 4.5 hours, to prepare 3-methylindole at a reaction yield of 97.5%.

### COMPARATIVE EXAMPLE 2

Under the hydrogen atmosphere, 8 g of indole-3-carboxaldehyde, 30 g of diglyme and 0.8 g of copper chromite catalyst (CuO content: 33 wt%) were introduced into a batch-type reactor, and reacted at 45 °C under a hydrogen pressure of 50 psig for 4 hours. The conversion of indole-3-carboxaldehyde was almost not observed.

Fig. 1 shows each of the reaction yields of the products obtained by Comparative Example 1 and Examples 1-8. As shown in Fig. 1, the copper or copper oxide-based catalyst used in Examples shows at least equal performance for the reduction of indole-3-carboxaldehyde when compared with Comparative Example 1, which indicates that the production cost may be reduced in a considerable extent. In particular, the reduced copper-based catalysts show higher performance for the reduction of indole-3-carboxaldehyde than the copper oxide-based catalyst as in Examples 7 and 8. Further, it is confirmed that the spent copper or copper oxide-based catalyst is capable of being readily regenerated and the performance thereof is as high as that of the fresh one, as shown in Example 4. It was also observed that the reaction yield hardly varies with sulfur content.

### INDUSTRIAL APPLICABILITY

As described above, according to the preparation method of the present invention, 3-methylindole compounds of high yields can be prepared from indole-3-carboxaldehyde derivatives through an environmentally safe and economical process using an inexpensive and reusable hydrogenation catalyst, without intended addition of a sulfur component.

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

## Claims

1. A method for preparing 3-methylindole derivatives, in which indole-3-carboxaldehyde derivatives having the following formula 1 are subjected to reduction in solvent using hydrogenation catalyst under the condition of a temperature of 50-300 °C and a pressure of 50-1,000 psig with hydrogen atmosphere to give 3-methylindole derivatives having the following formula 2, the hydrogenation catalyst comprising copper or copper oxide and inorganic oxide selected from the group consisting of silica, alumina, zirconia, titania, zinc oxide, chromium oxide, and combinations thereof: 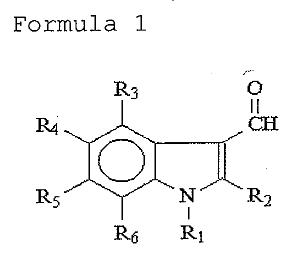 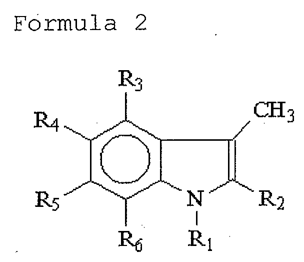 wherein R₁, R₂, R₃, R₄, R₅ and R₆, which are the same or different, each represents a hydrogen atom, a halogen atom, a hydroxy group, an alkyl group of 1-6 carbon atoms, an aryl group of 6-12 carbon atoms, or an alkoxy group of 1-12 carbon atoms.

2. The method as defined in claim 1, wherein the catalyst is used in the amount of 0.5-200 parts by weight based on 100 parts by weight of the indole-3-carboxaldehyde derivatives.

3. The method as defined in claim 1, wherein the copper content in the catalyst ranges from 3 to 50 wt%.

4. The method as defined in claim 1, wherein R₁, R₂, R₃, R₄, R₅ and R₆ are hydrogen atom, respectively.

5. The method as defined in claim 1, wherein the solvent is a polar solvent selected from the group consisting of ether, alcohol and mixtures thereof.

6. The method as defined in claim 5, wherein the ether is diglyme or dioxane, and the alcohol is methanol, ethanol or propanol.

7. The method as defined in claim 1, wherein the reduction step is performed at 80-250 °C.

8. The method as defined in claim 7, wherein the reduction step is performed under 100-700 psig.

9. The method as defined in claim 1, wherein said hydrogenation catalyst is used in the reduced form thereof.
